(19) Europäisches Patentamt
European Patent Office
Office européen des brevets

(11) Veröffentlichungsnummer: **0 130 366**
**B1**

# (12) EUROPÄISCHE PATENTSCHRIFT

(45) Veröffentlichungstag der Patentschrift: **30.01.91**

(21) Anmeldenummer: **84106113.8**

(22) Anmeldetag: **29.05.84**

(51) Int. Cl.$^5$: **C 07 D 233/56,**
C 07 D 249/08, A 01 N 43/50,
A 01 N 43/64

## (54) Arylalkylimidazolium- und -triazoliumsalze, Verfahren zu ihrer Herstellung und ihre Verwendung als Mikrozide.

(30) Priorität: **01.06.83 DE 3319845**

(43) Veröffentlichungstag der Anmeldung:
**09.01.85 Patentblatt 85/02**

(45) Bekanntmachung des Hinweises auf die Patenterteilung:
**30.01.91 Patentblatt 91/05**

(84) Benannte Vertragsstaaten:
**AT BE CH DE FR GB IT LI NL**

(56) Entgegenhaltungen:
**EP-A-0 000 017**
**EP-A-0 025 882**
**FR-A-2 329 657**

**Burger: Med. Chemistry, Third Bd., Part I, p. 74-75**

(73) Patentinhaber: **BASF Aktiengesellschaft**
**Carl-Bosch-Strasse 38**
**D-6700 Ludwigshafen (DE)**

(72) Erfinder: **Rentzea, Costin, Dr.**
**Richard Kuhn-Str. 1-3**
**D-6900 Heidelberg (DE)**
Erfinder: **Buschmann, Ernst, Dr.**
**Georg Ludwig Krebs-Str. 10**
**D-6700 Ludwigshafen (DE)**
Erfinder: **Sauter, Hubert, Dr.**
**Neckarpromenade**
**D-6800 Mannheim 1 (DE)**
Erfinder: **Ammermann, Eberhard, Dr.**
**Sachsenstr. 3**
**D-6700 Ludwigshafen (DE)**
Erfinder: **Pommer, Ernst-Heinrich, Dr.**
**Berliner Platz 7**
**D-6703 Limburgerhof (DE)**

**EP 0 130 366 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue Arylalkylimidazolium- und -triazoliumsalze, Verfahren zu ihrer Herstellung und mikrozide, d.h. fungizide und bakterizide Mittel, die diese Verbindungen als Wirkstoffe enthalten.

Es ist bekannt, N-Trichlormethylthio-tetrahydrophthalimid als Fungizid zu verwenden (Chemical Week, June 21, 1972, Seite 46). Seine Wirksamkeit befriedigt nicht in allen Fällen.

Es ist ferner bekannt, 1-(2-Phenyläthyl)-triazolium-Salze (EP—A—17) oder quaternäre Imidazoliumsalze (FR—A—2 329 657) als Fungizide zu verwenden. Diese Verbindungen unterscheiden sich von den neuen Verbindungen in ihrer chemischen Struktur.

Es wurde nun gefunden, daß Arylalkylimidazolium- und -triazoliumsalze der Formel

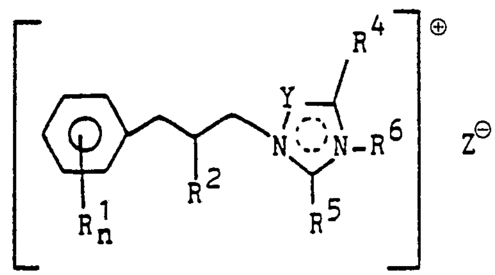

I

in der

$R^1$ $C_1$—$C_8$-Alkyl, halogensubstituiertes $C_1$—$C_4$-Alkyl, Phenyl, Halogenphenyl, Benzyl, Halogenbenzyl, Phenylethyl, Cyclohexyl, Acetyl, Propionyl, Benzoyl oder Halogen,

n 0, 1, 2 oder 3,

$R^2$ $C_1$—$C_4$-Alkyl oder $C_3$—$C_4$-Alkenyl,

$R^4$ und $R^5$ unabhängig voneinander Wasserstoff oder Methyl, Ethyl bedeuten,

Y N oder CH—$R^3$ bedeutet,

$R^3$ Wasserstoff oder Methyl, Ethyl bedeutet,

$R^6$ einen durch $C_{1-2}$-Alkoxy oder Halogen substituierten $C_{1-6}$-Alkyl- oder $C_{3-6}$-Alkenylrest oder einen $C_{1-6}$-Alkyl-, $C_{2-6}$-Alkenyl- oder $C_{3-4}$-Alkinylrest oder eine der Gruppen

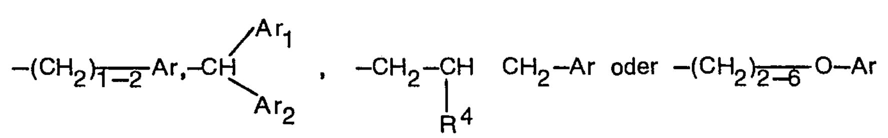

bedeutet, wobei Ar für 1- und 2-Naphthyl, Biphenylyl oder Phenyl steht und der Phenylrest durch Fluor, Chlor, Brom, Nitro, Trifluormethyl, Cyan oder $C_1$—$C_{13}$-Alkyl, $C_2$—$C_4$-Alkenyl oder $C_1$—$C_2$-Alkoxy substituiert sein kann und

Z ein Anion bedeutet, das ein Äquivalent einer nicht phytotoxischen Säure ist, eine gute mikrozide, fungizide und bakterizide Wirksamkeit zeigen.

$R^1$ bedeutet beispielsweise Wasserstoff, Fluor, Chlor, Brom, $C_1$—$C_8$-Alkyl, z.B. Methyl, Ethyl, n-Propyl, Isopropyl, tert.-Butyl, n-Pentyl, tert.-Amyl, 1,1-Dimethylbutyl, 1,1-Dimethylpentyl, 1,1-Dimethylhexyl, 1,1-Diethylpropyl, 1,1,2-Trimethylpropyl, Halogen-$C_1$—$C_4$-Alkyl z.B. 2-Chlor-1,1,-dimethylethyl, Trifluormethyl, Cyclohexyl, Phenyl, Halogenphenyl z.B. 4-Chlorphenyl, Benzyl, Halogenbenzyl z.B. 4-Chlorbenzyl, Phenylethyl, Acetyl, Propionyl oder Benzoyl;

$R^2$ bedeutet beispielsweise $C_1$—$C_4$-Alkyl, Methyl, Ethyl, Propyl, Butyl, $C_3$—$C_4$-Alkenyl, Allyl;

für $R^3$, $R^4$ und $R^5$ kommen Wasserstoff, Methyl oder Ethyl in Betracht.

$R^6$ bedeutet z.B. $C_1$—$C_6$-Alkyl, $C_2$—$C_6$-Alkenyl oder $C_3$—$C_4$-Alkinyl oder $C_1$—$C_6$-Alkyl oder $C_3$—$C_6$-Alkenyl, die durch $C_1$—$C_2$-Alkoxy oder Halogen substituiert sind, beispielsweise Methyl, Ethyl, n-Propyl, n-Butyl, Isobutyl, n-Pentyl, 4-Chlorbutyl, 2-Ethoxyethyl, n-Hexyl, Allyl, 2-Chlorallyl, 3-Chlorallyl, 2-Bromallyl, 3-Chlor-2-buten-1-yl, 3-Buten-1-yl, 3-Methyl-2-buten-1-yl, 2-Buten-1-yl, Propargyl oder die Reste

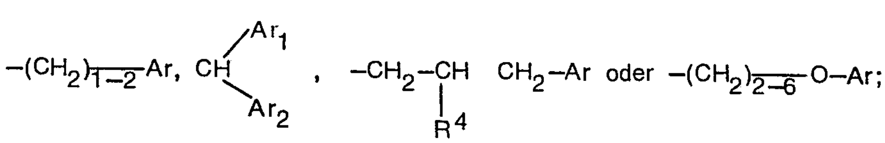

Ar, $Ar_1$ und $Ar_2$ bedeutet beispielsweise 4-Fluorphenyl, 3- und 4-Chlorphenyl, 4-Bromphenyl, 3,4-Dichlorphenyl, 2,4-Dichlorphenyl, 2,6-Dichlorphenyl, 4-Methoxyphenyl, 4-Ethoxyphenyl, 4-Methylphenyl, 4-Isopropylphenyl, 4-tert.-Butylphenyl, 4-Nitrophenyl, 4-Trifluormethylphenyl, 2,4-Dimethylphenyl und 4-Cyanphenyl;

Z bedeutet ein Anion, das ein Äquivalent einer nicht phytotoxischen Säure ist, beispielsweise Salzsäure, Bromwasserstoffsäure, Phenylsulfonsäure, p-Methylphenylsulfonsäure oder ein Äquivalent des Schwefelsäure-Anions.

Man erhält die Arylalkylimidazolium- und -triazoliumsalze der Formel I beispielsweise durch Umsetzung von

a) Verbindungen der Formel

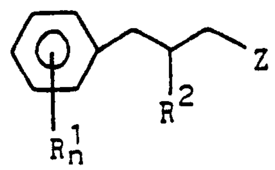

II

in der $R^1$, $R^2$, Z und n die oben angegebenen Bedeutungen haben, mit einem Imidazol- oder Triazol-Derivat der Formel

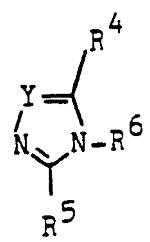

III

in der $R^4$, $R^5$, $R^6$ und Y die oben angegebenen Bedeutungen haben, oder

b) Verbindungen der Formel

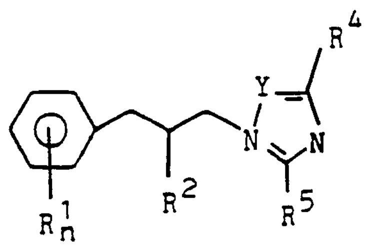

IV

in der $R^1$, $R^2$, $R^4$, $R^5$, Y und n die oben angegebenen Bedeutungen haben, mit einer Verbindung der Formel $R^6$—Z, in der $R^6$ und Z die oben angegebenen Bedeutungen haben.

Die Reaktionen a) und b) werden, gegebenenfalls in Gegenwart eines inerten Lösungs- oder Verdünnungsmittels, bei einer Temperatur im Bereich zwischen 20 und 150°C, vorzugsweise zwischen 30 und 140°C, durchgeführt. Der Ausgangsstoff der Formel II wird zweckmäßig mit einem bis zu 10-fachen molaren Überschuß des Imidazolderivates der Formel III umgesetzt.

Als bevorzugte, gegenüber den Reaktionsteilnehmern inerte Lösungs- oder Verdünnungsmittel können beispielsweise aliphatische oder aromatische, gegebenenfalls halogenierte Kohlenwasserstoffe, wie Pentan, Cyclohexan, Heptan, Benzol, Toluol, Xylol, Chlorbenzol, Dichlorbenzole, aliphatische Ketone, wie Aceton, Methylethylketon, Diethylketon oder Cyclopentanon, Ether wie Diethylether, Methyl-tert.-butylether, Dimethoxyethan, Tetrahydrofuran oder Dioxan, Ester wie Essigsäureethylester, Nitrile, wie Acetonitril, Amide, wie Dimethylformamid, Dimethylacetamid und N-Methylpyrrolidon, oder Gemische dieser Lösungsmittel verwendet werden.

Die Ausgangsstoffe der Formel II und IV sind bekannt (DE—OS 2 935 452). Imidazole oder Triazole der Formel III sind bekannt und sind größtenteils kommerziell zugänglich.

Die neuen Arylalkylimidazolium- und -triazoliumsalze der Formel I besitzen, sofern $R^2$ nicht Wasserstoff bedeutet, ein chirales, mit dem Ligand $R^2$ gebundenes Kohlenstoffatom und gegebenenfalls noch weitere Chiralitätszentren in $R^6$. Nach üblichen Methoden können die optisch reinen Enantiomeren bzw. die Diastereomeren erhalten werden. Die vorliegende Erfindung erfaßt sowohl diese Verbindungen in reiner Form als auch ihre Mischungen. Als Fungizide und Bakterizide sind sowohl die reinen Enantiomeren bzw. die einheitlichen Diastereomeren als auch die bei der Synthese üblicherweise anfallenden Mischungen wirksam.

Die folgenden Beispiele erläutern die Herstellung der neuen Verbindungen:

Beispiel 1

Die Lösung von 449 g (2 Mol) 1-Chlor-3-(4-tert.-butylphenyl)-2-methylpropan in 500 ml Dimethylformamid wurde mit 408 g (6 Mol) Imidazol versetzt und 10 Stunden bei 120°C nachgerührt. Man

ließ abkühlen und destillierte im Vakuum einer Wasserstrahlpumpe ab. Der Rückstand wurde in 1,5 l Methylenchlorid gelöst und viermal mit je 200 ml Wasser gewaschen. Die organische Schicht wurde abgetrennt, über $Na_2SO_4$ getrocknet und eingedampft. Die Destillation des Rohproduktes ergab 315,3 g (61,6% d. Th.) N-[3-(4-tert.-Butylphenyl)-2-methylpropyl]-imidazol vom Sdp. 170 bis 172°C, 0,5 mbar und $n_D^{28}$ = 1,5308.

Die Lösung von 10 g (0,039 Mol) N-[3-(4-tert.-Butylphenyl)-2-methylpropyl]-imidazol und 9,4 g (0,055 Mol) Benzylbromid in 20 ml trockenem Dioxan und 40 ml trockenem Acetonitril wurde 48 Stunden bei 70°C gerührt. Nach dem Abkühlen auf +5°C (Eisbad) wurde der kristalline Niederschlag abgesaugt, mit 30 ml Diethylether und schließlich mit 50 ml Pentan gewaschen und getrocknet.

Man erhält 8,7 g (52,2% d.Th.) $N^1$-Benzyl-$N^3$-[3-(4-tert.-butylphenyl)-2-methylpropyl]-imidazoliumbromid (Verbindung Nr. 1) vom Schmp. 140 bis 143°C.

Entsprechend Beispiel 1 lassen sich beispielsweise folgende Verbindungen der Formel I herstellen:

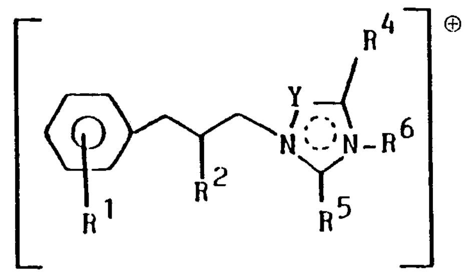 $Z^{\ominus}$ I

| Nr. | $R^1$ | $R^2$ | $R^3$ | $R^4$ | $R^5$ | $R^6$ | Y | Z | Fp [°C] |
|---|---|---|---|---|---|---|---|---|---|
| 2 | 4-tert.-$C_4H_9$ | $CH_3$ | H | H | H | $-CH_2-CH=CH_2$ | CH | Br | Harz |
| 3 | " | $CH_3$ | - | H | H | $-CH_2-CH=CH_2$ | N | Br | 144 - 146 |
| 4 | " | $CH_3$ | H | H | H | Propargyl | CH | Br | 156 - 158 |
| 5 | " | $CH_3$ | H | H | H | $-CH_2-CH=CH-Cl$ | CH | Cl | Harz |
| 7 | " | $CH_3$ | - | H | H | $-CH_2-CH=CH-Cl$ | N | Cl | 108 - 110 |
| 8 | " | $CH_3$ | H | H | H | $-CH_2-CH=CH-CH_3$ | CH | Br | Harz |
| 9 | " | $CH_3$ | - | H | H | $-CH_2-CH=CH-CH_3$ | N | Br | 146 - 148 |
| 10 | " | $CH_3$ | H | H | H | $-CH_2-C(Br)=CH_2$ | CH | Br | 134 - 136 |
| 11 | " | $CH_3$ | - | H | H | $-CH_2-C(Br)=CH_2$ | N | Br | 128 - 130 |
| 12 | " | $CH_3$ | H | H | H | $-CH_2C(Cl)=CH_2$ | CH | Cl | Harz |
| 13 | " | $CH_3$ | H | H | H | $-CH_2-CH=C(Cl)-CH_3$ | CH | Cl | Harz |
| 14 | " | $CH_3$ | H | H | H | $-CH_2-CH=CH-CH_2Cl$ | CH | Cl | Harz |
| 15 | 4-tert.-$C_4H_9$ | $CH_3$ | H | H | H | $-CH_2-C(Cl)=CCl_2$ | CH | Cl | 124 - 126 |
| 16 | 2,3,4-$Cl_3$ | n-$C_4H_9$ | H | H | H | $C_6H_5-CH_2-$ | CH | Br | 178 |
| 17 | 4-$(CH_3)_2CH-C(CH_3)_2$ | $CH_3$ | H | H | H | $C_6H_5-CH_2-$ | CH | Br | 117 |
| 18 | 4-tert.-$C_4H_9$ | $CH_3$ | - | H | H | $C_6H_5-CH_2-$ | N | Br | 180 - 183 |

| Nr. | $R^1$ | $R^2$ | $R^3$ | $R^4$ | $R^5$ | $R^6$ | Y | Z | Fp [°C] |
|---|---|---|---|---|---|---|---|---|---|
| 19 | " | $CH_3$ | H | H | H | 4 $F-C_6H_4-CH_2-$ | CH | Br | 138 - 141 |
| 20 | 2,3,4-$Cl_3$ | n-$C_4H_9$ | H | H | H | 4 $Cl-C_6H_4-CH_2-$ | CH | Br | 162 |
| 21 | 2,4-$Cl_2$ | $CH_3$ | H | H | H | 4 $Cl-C_6H_4-CH_2-$ | CH | Br | 125 |
| 22 | 4-tert.-$C_4H_9$ | $CH_3$ | H | H | H | 4 $Cl-C_6H_4-CH_2-$ | CH | Br | 177 - 178 |
| 23 | 4-tert.-$C_5H_{11}$ | $CH_3$ | H | H | H | 4-$Cl-C_6H_4-CH_2-$ | CH | Br | 161 |
| 24 | 4-$(CH_3)_2CH-C(CH_3)_2-$ | $CH_3$ | H | H | H | 4 $Cl-C_6H_4-CH_2-$ | CH | Br | 166 - 168 |
| 25 | 4-Cyclohexyl- | $CH_3$ | H | H | H | 4 $Cl-C_6H_4-CH_2-$ | CH | Br | 145 - 146 |
| 26 | 4-tert.-$C_4H_9$ | $CH_3$ | H | H | $-CH_3$ | 4 $Cl-C_6H_4-CH_2-$ | CH | Cl | 161 - 163 |
| 27 | " | $CH_3$ | H | H | H | 4 $Br-C_6H_4-CH_2-$ | CH | Br | 183 - 186 |
| 28 | 4-Cyclohexyl | $CH_3$ | H | H | H | 4-$Br-C_6H_4-CH_2-$ | CH | Br | 157 - 159 |
| 29 | 4-tert.-$C_4H_9$ | $CH_3$ | H | H | H | 4 $CH_3-C_6H_4-CH_2-$ | CH | Br | 136 - 139 |
| 30 | " | $CH_3$ | H | H | H | 4(tert.-$C_4H_9$)$C_6H_4-CH_2-$ | CH | Cl | 184 - 187 |
| 31 | 4-tert.-$C_4H_9$ | $CH_3$ | H | H | $CH_3$ | 4(tert.-$C_4H_9$)$C_6H_4-CH_2-$ | CH | Cl | 210 - 212 |
| 32 | " | $CH_3$ | $CH_3$ | $CH_3$ | $CH_3$ | 4(tert.-$C_4H_9$)$C_6H_4-CH_2-$ | CH | Cl | 184 - 187 |
| 33 | " | $CH_3$ | - | H | H | 4(tert.-$C_4H_9$)$C_6H_4-CH_2-$ | N | Br | 217 - 220 |
| 34 | " | $CH_3$ | H | H | H | 4(n-$C_{12}H_{25}$)$C_6H_4-CH_2-$ | CH | Cl | Harz |
| 35 | " | $CH_3$ | H | H | H | 2,4-$Cl_2C_6H_3-CH_2-$ | CH | Cl | 157 - 159 |
| 36 | " | $CH_3$ | - | H | H | 2,4-$Cl_2C_6H_3-CH_2-$ | N | Cl | 151 - 153 |
| 37 | " | $CH_3$ | H | H | H | 4-$CF_3C_6H_4-CH_2-$ | CH | Br | 182 - 184 |
| 38 | " | $CH_3$ | H | H | H | 4-$NO_2C_6H_4-CH_2-$ | CH | Br | 147 - 150 |
| 39 | " | $CH_3$ | H | H | H | 4-$CNC_6H_4-CH_2-$ | CH | Br | 98 - 102 |
| 40 | " | $CH_3$ | H | H | H | 2-$C_{10}H_7-CH_2-$ | CH | Cl | 183 - 188 |
| 41 | " | $CH_3$ | H | H | H | $(C_6H_5)_2CH-$ | CH | Br | Harz |

| Nr. | $R^1$ | $R^2$ | $R^3$ | $R^4$ | $R^5$ | $R^6$ | Y | Z | Fp [°C] |
|---|---|---|---|---|---|---|---|---|---|
| 42 | " | $CH_3$ | H | H | H | $(C_6H_5)_2CH-$ | CH | Br | 140 - 143 |
| 43 | " | $CH_3$ | H | H | H | $(4-ClC_6H_4)(C_6H_5)CH-$ | CH | Cl | Harz |
| 44 | " | $CH_3$ | H | H | H | $C_6H_5-CH_2-CH_2-$ | CH | Br | 109 - 112 |
| 46 | 4-tert.-$C_4H_9$ | $CH_3$ | - | H | H | $C_6H_5-CH_2-CH_2-$ | N | Br | 177 - 180 |
| 47 | " | $CH_3$ | H | H | H | $C_6H_5-CH=CH-CH_2-$ | CH | Br | 122 - 125 |
| 48 | " | $CH_3$ | H | H | H | 4-(tert.-$C_4H_9$)$C_6H_4$-<br>$-CH_2-CH(CH_3)-CH_2-$ | CH | Cl | 131 - 136 |
| 49 | " | $CH_3$ | H | H | $CH_3$ | 4-(tert.-$C_4H_9$)$C_6H_4$-<br>$-CH_2-CH(CH_3)-CH_2-$ | CH | Cl | 230 - 233 |
| 50 | " | $CH_3$ | H | H | H | $2-C_{10}H_7-O-CH_2-CH_2-$ | CH | Br | 110 - 112 |
| 51 | " | $CH_3$ | H | H | H | $C_6H_5-O-CH_2-CH_2-$ | CH | Br | 120 - 123 |
| 52 | " | $CH_3$ | H | H | H | $C_6H_5-O-CH_2-CH_2-CH_2-$ | CH | Br | Harz |
| 53 | " | $CH_3$ | H | H | H | $C_6H_5-O-(CH_2)_4-$ | CH | Br | Harz |
| 55 | " | $CH_3$ | H | H | H | $4-FC_6H_4-O-CH_2-CH_2-$ | CH | Br | 121 - 123 |
| 56 | " | $CH_3$ | H | H | H | $3-CH_3C_6H_4-O-CH_2-CH_2-$ | CH | Br | 104 - 106 |
| 57 | " | $CH_3$ | H | H | H | $2,4-Cl_2C_6H_3-O-CH_2-CH_2-$ | CH | Br | 131 - 134 |
| 58 | " | $CH_3$ | H | H | H | $2,4,6-(CH_3)_3C_6H_2-O-$<br>$-CH_2-CH_2-$ | CH | Br | 104 - 107 |
| 59 | " | $CH_3$ | H | H | H | $4-ClC_6H_4-O-(CH_2)_4-$ | CH | Br | Harz |
| 60 | " | $CH_3$ | H | H | $CH_3$ | $4-ClC_6H_4-O-(CH_2)_4-$ | CH | Br | Harz |
| 61 | " | $CH_3$ | - | H | H | $4-ClC_6H_4-O-(CH_2)_4-$ | N | Br | 139 - 141 |

Die neuen Wirkstoffe zeigen eine starke fungitoxische Wirksamkeit gegen phytopathogene Pilze. Sie dienen insbesondere zur Verhütung und Heilung von Pflanzenkrankheiten, die durch Mikroorganismen verursacht werden, wie z.B. Botrytis cinerea, Plasmopara viticola, Monilia fructigena, Alternaria solani, Sclerotinia sclerotiorum, Pyricularia oryzae, Pellicularia filamentosa, Erysiphe graminis, Erysiphe cichoracearum, Chaetomium globosum, Sclerotinia cinerea, Aspergillus niger, Xanthomonas oryzae, Xanthomonas citri, Erwinia amylovora, Phytophthora infestans (an Kartoffeln und Tomaten).

Die Wirkstoffe können gleichzeitig das Wachstum von zwei oder mehr der genannten Pilze unterdrücken und besitzen eine hohe Pflanzenverträglichkeit. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen.

Die fungiziden oder bakteriziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 bis 5 kg Wirkstoff je ha.

Die Wirkstoffe können auch zusammen mit anderen Wirkstoffen, z.B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten.

Die neuen Wirkstoffe werden beispielsweise in Form von direkt versprühbaren Lösungen, Suspensionen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiele für solche Pflanzenschutzmittel-Zubereitungen sind:

I. Man vermischt 90 Gewichtsteile der Verbindung des Beispiels 1 mit 100 Gewichtsteilen N-Methylpyrrolidon und erhält eine Lösung, die zur Anwendung in Form kleinster Tropfen geeignet ist.

II. 20 Gewichtsteile der Verbindung 16 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Doedecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100.000 Gewichtsteilen Wasser erhält man eine wäßrige Dispersion, die 0,02 Gew.-% des Wirkstoffs enthält.

III. 20 Gewichtsteile der Verbindung 19 werden in einer Mischung gelöst, die aus 30 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und Verrühren der Lösung in 100.000 Gewichtsteilen Wasser erhält man eine wäßrige Dispersion, die 0,02 Gew.% des Wirkstoffs enthält.

IV. 20 Gewichtsteile der Verbindung 14 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und Verrühren der Lösung in 100.000 Gewichtsteilen Wasser, erhält man eine wäßrige Dispersion, die 0,02 Gew.% des Wirkstoffs enthält.

V. 20 Gewichtsteile der Verbindung 20 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20.000 Gewichtsteilen Wasser erhält man eine Spritzbrühe, die 0,1 Gew.-% des Wirkstoffs enthält.

VI. 5 Gewichtsteile der Verbindung 22 werden mit 95 Gewichtsteilen feinteiligem Kaolin innig vermischt. Man erhält auf diese Weise ein Stäubemittel, das 5 Gew.-% des Wirkstoffs enthält.

VII. 30 Gewichtsteile der Verbindung 26 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung 28 werden mit 30 Teilen Natriumsalz eines Phenolsulfonsäure-Harnstoff-Formaldehyd-Kondensates, 2 Teilen Kieselgel und 48 Teilen Wasser innig vermischt. Man erhält eine stabile wäßrige Dispersion.

IX. 20 Teile der Verbindung 30 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Auch für technische Zwecke, z.B. als Holzschutzmittel, sind die neuen Wirkstoffe geeignet. Über die fungizide Wirkung hinaus wurde auch eine bakterizide Wirksamkeit der Verbindungen festgestellt, so daß sie auch als solche im Pflanzenschutz und als technische Mikrozide in Betracht kommen. Beispielsweise können folgende Mikroorganismen damit bekämpft werden.

Staphylococcus aureus, Escherichia coli, Klebsiella pneumoniae, Citrobacter freundii, Proteus vulgaris, Pseudomonas aeruginosa, Pseudomonas fluorescens, Pseudomonas lacrymans, Xanthomonas vesicatoria, Xanthomonas malvaccarum, Erwinia carotovora, Erwinia amylovora, Desulfovibrio desulfuricans, Streptoverticillium rubrireticuli, Aspergillus niger, Aspergillus versicolor, Penicillium funiculosum, Paecilomyces variotii, Trichoderma viride, Chaetomium globosum, Candida albicans, Geotrichum candidans, Monilia sitophila, Scenedesmus quadricauda, Chlorella vulgaris, Nostoc muscorum, Trichophyton mentagrophytis, Aureobasidium pullulaus, Sclerophoma pityophila, Comiophora puteana, Polystictus versicolor, Cladosporium herbarum, Alternaria tenuis.

Die üblichen Anwendungskonzentrationen betragen 0,01 bis 1% Wirkstoff, bezogen auf das Gewicht des zu schützenden Materials; beim Einsatz zur Wasserbehandlung bei der Erdölförderung, in Schwimmbädern, Rückkühlwerken, Luftbefeuchtungsanlagen, Blumenfrischhaltemitteln oder in der Papierindustrie sind Wirkstoffmengen von 5 bis 100 ppm ausreichend, um eine Mikroorganismenentwicklung zu unterdrücken. Gebrauchsfertige Mittel enthalten beispielsweise 0,2 bis 5% Wirkstoff.

Die folgenden Versuche belegen die biologische Wirkung der neuen Verbindungen. Vergleichsmittel ist stets der bekannte, insbesondere zur Bekämpfung von Botrytis geeignete Wirkstoff N-Trichlormethylthio-tetrahydrophthalimid (I) (Chem. Week, June 21, 1972, Seite 46).

Versuch 1

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80% Wirkstoff und 20% Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise die Verbindungen 12, 14, 16, 20, 21, 26, 30, 31, 32, 33, 34, 41, 42, 43, 48, 49, 50, 60 und 61 bei der Anwendung als 0,05 %ige Spritzbrühe eine bessere fungizide Wirkung (beispielsweise 97%) zeigen als die bekannte Verbindung I (beispielsweise 70%).

Versuch 2

Wirksamkeit gegen Phytophthora infestans an Tomaten

Blätter von Topfpflanzen der Sorte "Große Fleischtomate" werden mit wäßriger Spritzbrühe, die 80% Wirkstoff und 20% Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß beispielsweise die Verbindungen 1, 16, 19, 20, 21, 22, 24, 26, 27, 28, 29, 30, 31, 32, 33, 35, 36, 37, 38, 40, 43, 47, 48, 49, 50, 51, 55, 56, 57, 59 und 61 bei der Anwendung als 0,025 %ige Spritzbrühe eine bessere fungizide Wirkung (beispielsweise 97%) zeigen als die bekannte Verbindung I (beispielsweise 60%).

Versuch 3

Wirksamkeit gegen Plasmopara viticola

Blätter von Topfreben der Sorte "Muller-Thurgau" werden mit wäßriger Spritzbrühe, die 80% Wirkstoff und 20% Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß beispielsweise die Verbindungen 1, 20, 22, 23, 25, 27, 29, 30, 35, 37, 40, 43, 47, 51, 55, 56 und 58 bei der Anwendung als 0,025 %ige Spritzbrühe eine gute fungizide Wirkung (beispielsweise 90%) zeigen.

Versuch 4

Zur Ermittlung der Wirksamkeit der neuen Verbindungen gegenüber Bakterien werden zu je 5 ml steigender Verdünnung der Wirkstoffe 5 ml doppelt konzentrierter Nährbouillon in sterile Reagenzgläser gegeben und vermischt. Durch Zugabe von einem Tropfen einer 1:10 verdünnten 16 Stunden alten Bouillon-Kultur der Bakterien-Arten Staphylococcus aureus bzw. Escherichia coli oder Pseudomonas aeruginosa werden dann die Röhrchen beimpft und 24 Stunden bei 37°C bebrütet. Nach dieser Zeit werden Proben aus den den Röhrchen auf Bakteriennährböden übertragen und diese ebenfalls 24 Stunden lang bei 37°C bebrütet. Diejenige Verdünnungsstufe, bei welcher keine sichtbare Bakterienentwicklung mehr erfolgt, wird als minimale Hemmkonzentration angegeben.

Das Ergebnis des Versuches zeigt, daß beispielsweise die Verbindungen 1, 19, 22, 29, 30, 40, 41, 47, 50, 51, 55, 57 und 59 eine minimale Hemmkonzentration von 6 bis 200 Teilen (Gewichtsteile) Wirkstoff pro Million Teile Nährlösung zeigen.

Versuch 5

Zur Prüfung der Wirksamkeit gegenüber Pilzen werden die Wirkstoffe einer für das Wachstum der Pilze Aspergillus niger, Oidium lactis bzw. Candida albicans optimal geeigneten Nährlösung in Mengen von 100, 50, 25, 12, 6 und 3 Gewichtsteilen pro Million Teile Nährlösung zugesetzt. Jeweils 10 ml Nährlösungs-Wirkstoffgemisch werden in sterile Reagenzgläser gegeben und mit einem Tropfen einer Sporensuspension beimpft, die 106 Konidien bzw. Zellen enthält. Nach 120-stündiger Bebrütung wird die Verdünnungsstufe ermittelt, bei welcher kein sichtbares Wachstum der Pilze mehr erfolgt; sie wird in der Tabelle als minimale Hemmkonzentration angegeben.

Das Ergebnis des Versuches zeigt, daß beispielsweise die Verbindungen 1, 19, 22, 29, 30, 40, 41, 47, 50, 51, 55, 57 und 59 eine minimale Hemmkonzentration von 6 bis 200 Teilen Wirkstoff pro Million Teile Nährlösung zeigen.

**Patentansprüche**

1. Arylalkylimidazolium- und -triazoliumsalze der Formel

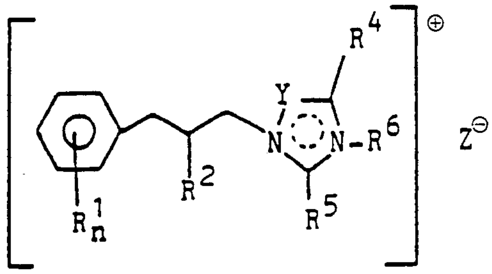

I,

in der

$R^1$ $C_1$—$C_8$-Alkyl, halogensubstituiertes $C_1$—$C_4$-Alkyl, Phenyl, Halogenphenyl, Benzyl, Halogenbenzyl, Phenylethyl, Cyclohexyl, Acetyl, Propionyl, Benzoyl oder Halogen,

n 0, 1, 2 oder 3,

$R^2$ $C_1$—$C_4$-Alkyl oder $C_3$—$C_4$-Alkenyl,

$R^4$ und $R^5$ unabhängig voneinander Wasserstoff oder Methyl, Ethyl bedeuten,

Y N oder CH—$R^3$ bedeutet,

$R^3$ Wasserstoff oder Methyl, Ethyl bedeutet,

$R^6$ einen durch $C_{1-2}$-Alkoxy oder Halogen substituierten $C_{1-6}$-Alkyl- oder $C_{3-6}$-Alkenylrest oder einen $C_{1-6}$-Alkyl-, $C_{2-6}$-Alkenyl- oder $C_{3-4}$-Alkinylrest oder eine der Gruppen

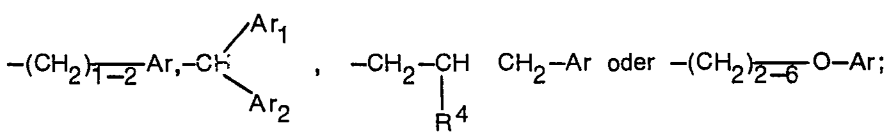

bedeutet, wobei Ar, $Ar_1$ und $Ar_2$ für 1- und 2-Naphthyl, Biphenyl oder Phenyl steht und der Phenylrest durch Fluor, Chlor, Brom, Nitro, Trifluormethyl, Cyan, $C_1$—$C_{13}$-Alkyl, $C_2$—$C_4$-Alkenyl oder $C_1$—$C_2$-Alkoxy substituiert sein kann und

Z ein Anion bedeutet, das ein Äquivalent einer nicht phytotoxischen Säure ist.

2. Salze der Formel I gemäß Anspruch 1, dadurch gekennzeichnet, daß

$R^1$ Chlor, Brom, $C_{1-8}$-Alkyl, Cyclohexyl, Benzyl oder Acetyl,

n 0, 1 oder 2,

$R^2$ $C_{1-4}$-Alkyl, $C_{3-4}$-Alkenyl,

Y N oder CH—$R^3$ bedeutet,

$R^3$ $R^4$ und $R^5$ unabhängig voneinander Wasserstoff, Methyl oder Ethyl,

$R^6$ Methyl, Ethyl, n-Propyl, Isopropyl, n-Butyl, Isobutyl, n-Pentyl, Isopentyl, n-Hexyl, Allyl, 2-Chlorpropenyl, 2-Brompropenyl, 3-Chlorpropenyl, 2-Buten-1-yl, 3-Methyl-2-buten-1-yl, 2-Methylpropenyl, 3-Chlor-2-buten-1-yl, Propargyl oder die Reste

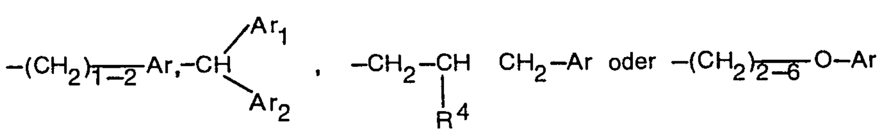

bedeutet, wobei Ar, $Ar_1$ und $Ar_2$ für 1- und 2-Naphtyl, Biphenyl, Phenyl, 4-Fluorphenyl, 3- und 4-Chlorphenyl, 4-Bromphenyl, 3,4-Dichlorphenyl, 2,4-Dichlorphenyl, 2,6-Dichlorphenyl, 2,4,6-Trichlorphenyl, 2-Methyl-4-chlorphenyl, 4-Methoxyphenyl, 4-Ethoxyphenyl, 4-Methylphenyl, 4-Nitrophenyl, 4-Isopropylphenyl, 4-tert.-Butylphenyl, 4-Dodecylphenyl, 4-Trifluormethylphenyl, 2-Trifluormethyl-4-chlorphenyl, 2,4-Dimethylphenyl, 2,4,6-Trimethylphenyl und 4-Cyanophenyl steht, und

Z ein Äquivalent des Anions einer nicht phytotoxischen Säure bedeutet.

3. Verfahren zur Herstellung von Arylalkylimidazolium- und -triazoliumsalzen gemäß Anspruch 1, dadurch gekennzeichnet, daß man

a) Verbindungen der Formel

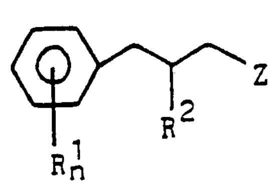

II,

in der $R^1$, $R^2$, Z und n die im Anspruch 1 angegebenen Bedeutungen haben, mit einem Imidazol- oder Triazol-Derivat der Formel

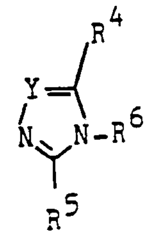

III,

in der $R^4$, $R^5$, $R^6$ und Y die im Anspruch 1 angegebenen Bedeutungen haben, oder

b) Verbindungen der Formel

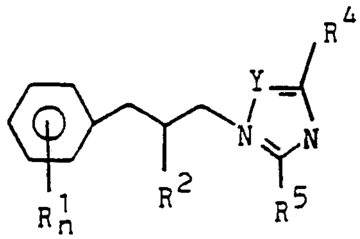

IV,

in der $R^1$, $R^2$, $R^4$, $R^5$, Y und n die im Anspruch 1 angegebenen Bedeutungen haben, mit einer Verbindung der Formel $R^6$—Z, in der $R^6$ und Z die im Anspruch 1 angegebenen Bedeutungen haben, umsetzt.

4. Mikrozides Mittel, enthaltend einen festen oder flüssigen Trägerstoff und ein Arylalkylimidazolium- oder -triazoliumsalz gemäß Anspruch 1.

5. Verfahren zur Bekämpfung von nicht humanpathogenen Pilzen und Bakterien, dadurch gekennzeichnet, daß man ein Arylalkylimidazolium- oder triazoliumsalz gemäß Anspruch 1 auf Pilze oder Bakterien oder auf durch Bakterien- oder Pilzbefall bedrohte Materialien, Flächen, Pflanzen oder Saatgüter einwirken läßt.

6. Verfahren zur Herstellung eines mikroziden Mittels gemäß Anspruch 4, dadurch gekennzeichnet, daß man ein Arylalkylimidazolium- oder -triazoliumsalz gemäß Anspruch 1 mit festen oder flüssigen Trägerstoffen sowie gegebenenfalls mit einem oder mehreren oberflächenaktiven Mitteln mischt.

7. Fungizides Mittel, enthaltend einen festen oder flüssigen Trägerstoff und ein Arylalkylimidazolium- oder -triazoliumsalz der Formel I gemäß Anspruch 1.

8. Bakterizides Mittel, enthaltend einen festen oder flüssigen Trägerstoff und ein Arylalkylimidazolium- oder -triazoliumsalz der Formel I gemäß Anspruch 1.

9. Fungizides Mittel, enthaltend einen festen oder flüssigen Trägerstoff und ein Arylalkylimidazolium- oder -triazoliumsalz gemäß Anspruch 2.

**Revendications**

1. Sels d'arylalkylimidazolium et -triazolium de formule

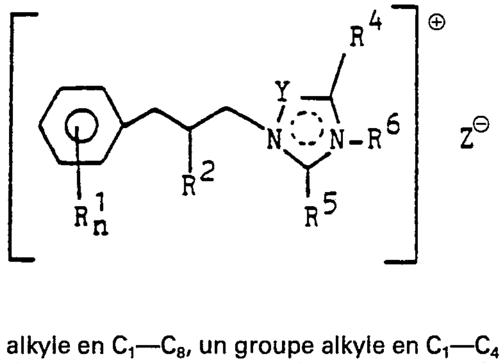

I,

dans laquelle

$R^1$ représente un groupe alkyle en $C_1$—$C_8$, un groupe alkyle en $C_1$—$C_4$ substitué par des halogènes, un groupe phényle, halogénophényle, benzyle, halogénobenzyle, phényléthyle, cyclohexyle, acétyle, propionyle, benzoyle ou un halogène,

n est égal à 0, 1, 2 ou 3,

$R^2$ représente un groupe alkyle en $C_1$—$C_4$ ou alcényle en $C_3$—$C_4$,

$R^4$ et $R^5$ représentent chacun, indépendamment l'un de l'autre, l'hydrogène, un groupe méthyle ou éthyle,

Y représente N ou CH—$R^3$,

$R^3$ représente l'hydrogène ou un groupe méthyle, éthyle,

$R^6$ représente un groupe alkyle en $C_1$—$C_6$ ou alcényle en $C_3$—$C_6$ substitué par un groupe alcoxy en $C_1$—$C_2$ ou des halogènes, ou un groupe alkyle en $C_1$—$C_6$, alcényle en $C_2$—$C_6$ ou alcynyle en $C_3$—$C_4$, ou l'un des groupes

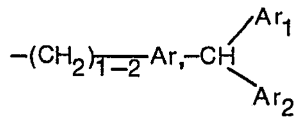 , 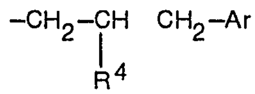 ou $-(CH_2)_{2-6}-O-Ar$

dans lesquels Ar, $Ar_1$ et $Ar_2$ représentent chacun un groupe 1- ou 2-naphtyle, biphényle ou phényle et le groupe phényle peut être substitué par le fluor, le chlore, le brome, des groupes nitro, trifluorométhyle, cyano, alkyle en $C_1$—$C_{13}$, alcényle en $C_2$—$C_4$ ou alcoxy en $C_1$—$C_2$, et

Z représente un anion correspondant à un équivalent d'un acide non phytotoxique.

2. Sels de formule I de la revendication 1, caractérisés en ce que:

$R^1$ représente le chlore, le brome, un groupe alkyle en $C_1$—$C_8$, cyclohexyle, benzyle ou acétyle,

n est égal à 0, 1 ou 2,

$R^2$ représente un groupe alkyle en $C_1$—$C_4$, alcényle en $C_3$—$C_4$,

Y représente N ou CH—$R^3$,

$R^3$ $R^4$ et $R^5$ représentent chacun, indépendamment les uns des autres, l'hydrogène, un groupe méthyle ou éthyle,

$R^6$ représente un groupe méthyle, éthyle, n-propyle, isopropyle, n-butyle, isobutyle, n-pentyle, isopentyle, n-hexyle, allyle, 2-chloropropényle, 2-bromopropényle, 3-chloropropényle, 2-butèn-1-yle, 3-méthyl-2-butèn-1-yle, 2-méthylpropényle, 3-chloro-2-butèn-1-yle, propargyle ou les groupes

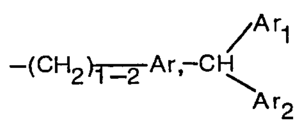 , 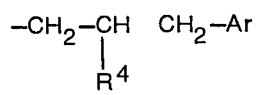 ou $-(CH_2)_{2-6}-O-Ar$;

dans lesquels Ar, $Ar_1$ et $Ar_2$ représentent chacun un groupe 1- ou 2-naphtyle, biphényle, phényle, 4-fluorophényle, 3- ou 4-chlorophényle, 4-bromophényle, 3,4-dichlorophényle, 2,4-dichlorophényle, 2,6-dichlorophényle, 2,4,6-trichlorophényle, 2-méthyl-4-chlorophényle, 4-méthoxyphényle, 4-éthoxyphényle, 4-méthylphényle, 4-nitrophényle, 4-isopropylphényle, 4-tert-butylphényle, 4-dodécyl-phényle, 4-trifluorométhylphényle, 2-trifluorométhyl-4-chlorophényle, 2,4-diméthylphényle, 2,4,6-triméthylphényle ou 4-cyanophényle, et

Z représente un équivalent de l'anion d'un acide non phytotoxique.

3. Procédé de préparation des sels d'arylalkylimidazolium ou -triazolium de la revendication 1, caractérisé en ce que:

a) on fait réagir des composés de formule

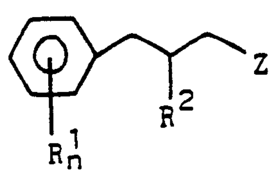

II,

dans laquelle $R^1$, $R^2$, Z et n ont les significations indiquées dans la revendication 1, avec un dérivé d'imidazole ou de triazole de formule

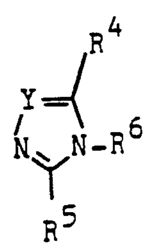

III,

dans laquelle $R^4$, $R^5$, $R^6$ et Y ont les significations indiquées dans la revendication 1, ou bien

b) on fait réagir des composés de formule

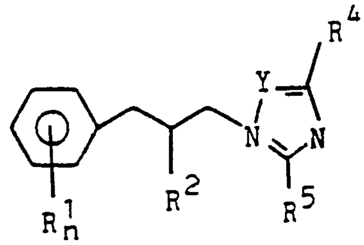

IV,

dans laquelle $R^1$, $R^2$, $R^4$, $R^5$, Y et n ont les significations indiquées dans la revendication 1, avec un composé de formule $R^6$—Z dans laquelle $R^6$ et Z ont les significations indiquées dans la revendication 1.

4. Produit microcide contenant un véhicule solide ou liquide et un sel d'arylalkylimidazolium ou -triazolium de la revendication 1.

5. Procédé pour combattre les mycètes et bactéries autres que les mycètes et bactéries pathogènes pour les humains, caractérisé en ce que l'on fait agir sur les mycètes ou bactéries ou sur les matériaux, surfaces, plantes ou semences menacés d'une attaque par des bactéries ou des mycètes, un sel d'arylalkylimidazolium ou -triazolium de la revendication 1.

6. Procédé de préparation d'un produit microcide selon la revendication 4, caractérisé en ce que l'on mélange un sel d'arylalkylimidazolium ou -triazolium de la revendication 1 avec des véhicules solides ou liquides et le cas échéant un ou plusieurs agents tensioactifs.

7. Produit fongicide contenant un véhicule solide ou liquide et un sel d'arylalkylimidazolium ou -triazolium de formule I de la revendication 1.

8. Produit bactéricide contenant un véhicule solide ou liquide et un sel d'arylalkylimidazolium ou -triazolium de formule I de la revendication 1.

9. Produit fongicide contenant un véhicule solide ou liquide et un sel d'arylalkylimidazolium ou -triazolium de la revendication 2.

## Claims

1. An arylalkylimidazolium or -triazolium salt of the formula

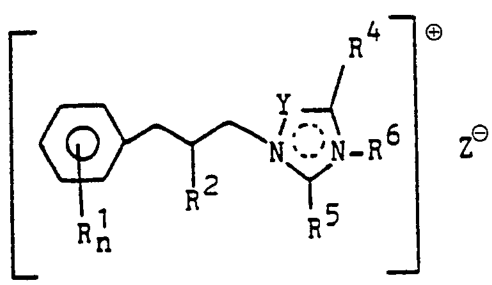

I,

where

$R^1$ is $C_1$—$C_8$-alkyl, halogen-substituted $C_1$—$C_4$-alkyl, phenyl, halophenyl, benzyl, halobenzyl, phenylethyl, cyclohexyl, acetyl, propionyl, benzoyl or halogen,

n is 0, 1, 2 or 3,

$R^2$ is $C_1$—$C_4$-alkyl or $C_3$—$C_4$-alkenyl,

$R^4$ and $R^5$ independently of one another are each hydrogen, methyl or ethyl,

Y is N or CH—$R^3$,

$R^3$ is hydrogen, methyl or ethyl,

$R^6$ is a $C_1$—$C_2$-alkoxy-substituted or halogen-substituted $C_1$—$C_6$-alkyl or $C_3$—$C_6$-alkenyl radical or is $C_1$—$C_6$-alkyl, $C_2$—$C_6$-alkenyl, $C_3$—$C_4$-alkynyl or one of the groups

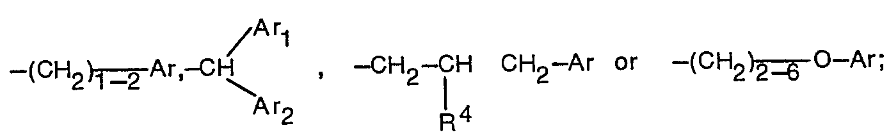

in which Ar, $Ar_1$ and $Ar_2$ are each 1- or 2-naphthyl, biphenyl or phenyl and the phenyl radical may be substituted by fluorine, chlorine, bromine, nitro, trifluoromethyl, cyano, $C_1$—$C_{13}$-alkyl, $C_2$—$C_4$-alkenyl or $C_1$—$C_2$-alkoxy, and

Z is an anion which is one equivalent of a nonphytotoxic acid.

2. A salt of the formula I as claimed in claim 1, wherein

$R^1$ is chlorine, bromine, $C_1$—$C_8$-alkyl, cyclohexyl, benzyl or acetyl,

n is 0, 1 or 2,

$R^2$ is $C_1$—$C_4$-alkyl or $C_3$—$C_4$-alkenyl,

Y is N or CH—$R^3$,

$R^3$, $R^4$ and $R^5$ independently of one another are each hydrogen, methyl or ethyl,

$R^6$ is methyl, ethyl, n-propyl, isopropyl, n-butyl, isobutyl, n-pentyl, isopentyl, n-hexyl, allyl, 2-chloropropenyl, 2-bromopropenyl, 3-chloropropenyl, 2-buten-1-yl, 3-methyl-2-buten-1-yl, 2-methylpropenyl, 3-chloro-2-buten-1-yl, propargyl or a radical

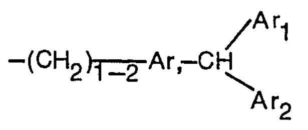 , 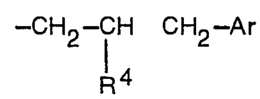 or $-(CH_2)_{2-6}-O-Ar$;

in which Ar, $Ar_1$ and $Ar_2$ are each 1- or 2-naphthyl, biphenyl, phenyl, 4-fluorophenyl, 3- or 4-chlorophenyl, 4-bromophenyl, 3,4-dichlorophenyl, 2,4-dichlorophenyl, 2,6-dichlorophenyl, 2,4,6-trichlorophenyl, 2-methyl-4-chlorophenyl, 4-methoxyphenyl, 4-ethoxyphenyl, 4-methylphenyl, 4-nitrophenyl, 4-isopropylphenyl, 4-tert.-butylphenyl, 4-dodecylphenyl, 4-trifluoromethylphenyl, 2-trifluoromethyl-4-chlorophenyl, 2,4-dimethylphenyl, 2,4,6-trimethylphenyl or 4-cyanophenyl, and

Z is one equivalent of the anion of a nonphytotoxic acid.

3. A process for the preparation of an arylalkylimidazolium or -triazolium salt as claimed in claim 1, wherein

a) a compound of the formula

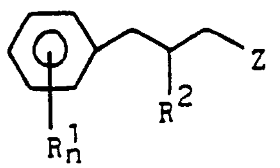 II,

where $R^1$, $R^2$, Z and n have the meanings stated in claim 1, is reacted with an imidazol or triazol derivative of the formula

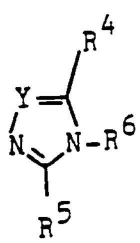 III,

where $R^4$, $R^5$, $R^6$ and Y have the meanings stated in claim 1, or

b) a compound of the formula

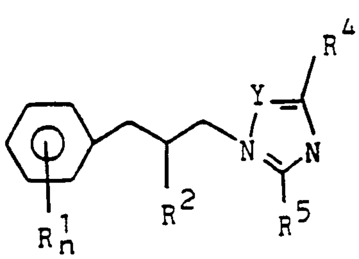 IV,

where $R^1$, $R^2$, $R^4$, $R^5$, Y and n have the meanings stated in claim 1, is reacted with a compound of the formula $R^6$—Z, where $R^6$ and Z have the meanings stated in claim 1.

4. A microcide, containing a solid or liquid carrier and an arylalkylimidazolium or -triazolium salt as claimed in claim 1.

5. A method for controlling fungi and bacteria which are not pathogenic to humans, wherein arylalkylimidazolium or -triazolium salt as claimed in claim 1 is allowed to act on fungi or bacteria or on materials, surfaces, plants or seeds threatened by bacterial or fungal attack.

6. A process for the preparation of a microcide as claimed in claim 4, wherein an arylalkylimidazolium

or -triazolium salt as claimed in claim 1 is mixed with solid or liquid carriers and, if required, with one or more surfactants.

7. A fungicide containing a solid or liquid carrier and an arylalkylimidazolium or -triazolium salt of the formula I as claimed in claim 1.

8. A bactericide containing a solid or liquid carrier and an arylalkylimidazolium or -triazolium salt of the formula I as claimed in claim 1.

9. A fungicide containing a solid or liquid carrier and an arylalkylimidazolium or -triazolium salt as claimed in claim 2.